# EUROPEAN PATENT APPLICATION

(11) **EP 3 249 039 A1**
(43) Date of publication of application: **29.11.2017**
(21) Application number: 16171339.1
(22) Date of filing: 25.05.2016
(51) Int. Cl.: C12M 3/00, A01N 1/02, A61L 27/36, C12M 1/00

(54) **PERFUSION-BIOREACTOR AND METHOD FOR PROCESSING VASCULARIZED COMPOSITE TISSUE**

(71) Applicant: Université Catholique de Louvain, 1348 Louvain-La-Neuve (BE)
(72) Inventor: Duisit, Jérôme, 1150 Brussel (BE); Herman, Benoît, 3080 Tervuren (BE); Gianello, Pierre, 1330 Rixensart (BE); Lengelé, Benoît, 1380 Lasne (BE)
(74) Representative: Gyi, Jeffrey Ivan

(57) **Abstract**

A bioreactor (100) for a vascularized structure (200) is provided comprising a vasculated portion (202) and a vascular pedicle (204), the bioreactor (100) comprising a first void space (112) configured to receive the vascular pedicle (204) optionally contained in a first vessel (110) having a first opening (114), a second void space (142) in fluid contact with at least part of the vasculated portion (202), and a support element (170) for supporting the vascularized structure (200) and fluidly separating the first void space (112) from the second void space (142), comprising an aperture region (176) comprising one or more apertures configured to receive at least part of the vascular pedicle (204).

## Description

### Field of the invention

Described herein is a perfusion-bioreactor for processing tissue and organs, in particular for a vascularized structure applicable especially in the field of tissue engineering.

### Background to the invention

Perfusion-Bioreactors are principally designed for *in vitro* perfusion and regeneration ((re)cellularization) by culturing cells in acellular organs scaffolds such as a heart, liver, kidney and the like for eventual transplantation into a subject. Existing bioreactors typically comprise a liquid vessel and a plurality of ports for exchange of perfusion fluids, however, they have been found to be unsuitable for body parts, such as vascularized composite tissue (VCT, or synonym Composite Tissue Allograft (CTA)), for instance those having multiple layers and interfaces, in particular a skin layer. There is a substantial need for processing VCT, such as limbs, trunk, face and scalp in its larger association, uterus or addressing single layers: simples like skin flap, muscle flap; or complex associations in targeted areas like subunits: ear, nose, lips, eyelids, tongue, fingers, breast, genitalia- for preservation, for perfusion decellularization/recellularization technology or cellularization of synthetic scaffolds. Decellularization is a process of removing native cells from a donor tissue with preserving its extracellular matrix (ECM) and associated biochemical components, generating matrix that can be repopulated (recellularized) with cells from the transplant recipient. This approach avoids or reduces the possibility of transplant rejection by the recipient. The present invention aims to provide a solution to the problem of vascularized composite tissue bioengineering.

### Summary of the invention

Described herein is a bioreactor (100) for a vascularized structure (200) comprising a vasculated portion (202) and a vascular pedicle (204), the bioreactor (100) comprising:
- a first void space (112) configured to receive the vascular pedicle (204) optionally contained in a first vessel (110) having a first opening (114),
- a second void space (142) in fluid contact with at least part of the vasculated portion (202),
- a support element (170) for supporting the vascularized structure (200) and fluidly separating the first void space (112) from the second void space (142), comprising an aperture region (176) comprising one or more apertures configured to receive at least part of the vascular pedicle (204).

The bioreactor (100) may be provided wherein:
- the support element (170) comprises a body provided with a first side (172) adjoining the first void space (112) and a second side (174) adjoining the second void space (142), and wherein the aperture region (176) connects the first side (172) with the second side (174),
- the second void space (142) is contained in a second vessel (140) having a second opening (144) or contained in a gap between the support element (170) and a covering plate.

The support element (170) may further comprise a clamp or more suture anchors configured to fixedly attach at least part of the vasculated portion (202) around a periphery of the aperture region (176) thereby fluidly sealing the aperture region (176).

The first (110) vessel and second vessel (140) or covering plate may be each dismountably attachable to the support element (170). They may be mutually fluidly isolated when the aperture region (176) is sealed.

The first vessel (110) may be further disposed with a sealable access opening (111), for manual access to the vascular pedicle (204).

The first vessel (110) may be further disposed with one or more ports (113) in fluid connection with the first void space (112) for inlet and/or outlet of fluid, of one or more electrically conducting cables, or of one or more flexible cords, optionally some ports being arranged at different distances from the first opening (114) or a different peripheral positions around first vessel.

The second vessel (140) may be further disposed with one or more ports (113) in fluid connection with the second void space (142) for inlet and/or outlet of fluid, of one or more electrically conducting cables, or of one or more flexible cords, optionally some ports on the second vessel (140) being arranged at different distances from the second opening (114) or a different peripheral positions around second vessel.

The support element (170) may be configured to position the aperture region (176) within the first void space (112), and optionally to provide a gap between a wall (118) of the first vessel (110) and the support element (170), optionally the gap having an annular form.

The bioreactor (100) may further comprise a third void space (192) separated from the first (112) or second (142) void space by the support element (170), configured to receive at least a part of the vasculated portion (202) not received by the received by the second void space (142).

Further provided is a use of a bioreactor (100) as described herein, for perfusion of a vascularized structure (200), for decellularization of a vascularized composite tissue, recellularization of a vascularized composite tissue scaffold or for preservation of vascularized composite tissue.

The use may be provided wherein:
- the first void space (112) is configured to contain liquid, and the second void space (142) is configured to contain a gaseous atmosphere or *vice versa,* or
- the first void space (112) and the second void space (142) are each configured to contain a liquid, or
- the first void space (112) and the second void space (142) are each configured to contain a gaseous environment.

Further provided is a method for perfusion of a vascularized structure (200), comprising the steps:
- providing a bioreactor (100) according to any of claims 1 to 9,
- attaching the vascularized structure (200) to the support element (170) such that at least part of the vasculated portion (202) is in fluid connection with the second void space (142) and at least part of the vascular pedicle (204) is disposed in the first void space (112),
- whereby a part of the vascularized structure (200) is sutured and/or clamped over the aperture region (176) to seal it, thereby isolating the first void space (112) from the second void space (142), and
- perfusing the vascularized structure (200) through vasculature of vascular pedicle (204).

Further provided is a kit comprising a bioreactor (100) according to any of claims 1 to 9, wherein there are at least two interchangeable support elements (170), each having a different aperture region (176) size and/or shape.

The kit may further comprise at least two different drawing templates, one for each different support element (170) for marking skin of a donor for harvesting such that the size and shape of the skin harvested is suitable for suturing and/or clamping over the corresponding aperture region (176) size and/or shape of the support element (170).

The vascularized structure (200) according to the use, the method according or the kit according may be a vascularized composite tissue or a vascularized composite tissue scaffold.

### Figure Legends

**FIG. 1** is a schematic illustration of a cross-section through a first vessel and second vessel of a bioreactor described herein.
**FIG. 2** panels **A** and **B** depict respectively plan and side views of a support element as described herein.
**FIG. 3** depicts a vascularized structure exemplified as a part of an arm.
**FIG. 4** illustrates an assembled bioreactor disposed with a vascularized structure.
**FIG. 5** illustrates cross-sectional view of a support element comprising a clamp mating body.
**FIG. 6** is a photograph of part of a bioreactor showing the second vessel disposed with the vasculated portion of vascularized structure that is a hand.
**FIG. 7** is a photograph of part of a bioreactor showing the first vessel disposed with the vascular pedicle of a vascularized structure that is a hand.
**FIG. 8** is an isometric view of an exemplary bioreactor described herein
**FIG. 9** is a schematic illustration of a cross-section through an exemplary bioreactor as described herein comprising three void spaces.
**FIG. 10** panels **A to D** depict different sizes of drawing template.
**FIG. 11** panel **A** depicts a vascularized structure that is a skin flap. Panel **B** depicts the vascularized structure that is the skin flap of panel **A** provided in a bioreactor.
**FIG. 12** panel **A** depicts a vascularized structure that is a face comprising a vasculated portion and a vascular pedicle. Panel **B** depicts the vascularized structure that is the face of panel **A** provided in a bioreactor.
**FIG. 13** depicts a vascularized structure that is a vagina and uterus comprising a vasculated portion (uterine, vaginal) and a vascular pedicle provided in a bioreactor
**FIG. 14** panel **A** depicts a vascularized structure that is an arm disposed in a bioreactor as shown in **FIG. 4**, together with an ancillary support structure that is a cylindrical cage and a further ancillary support structure that is hollow cylindrical projection.

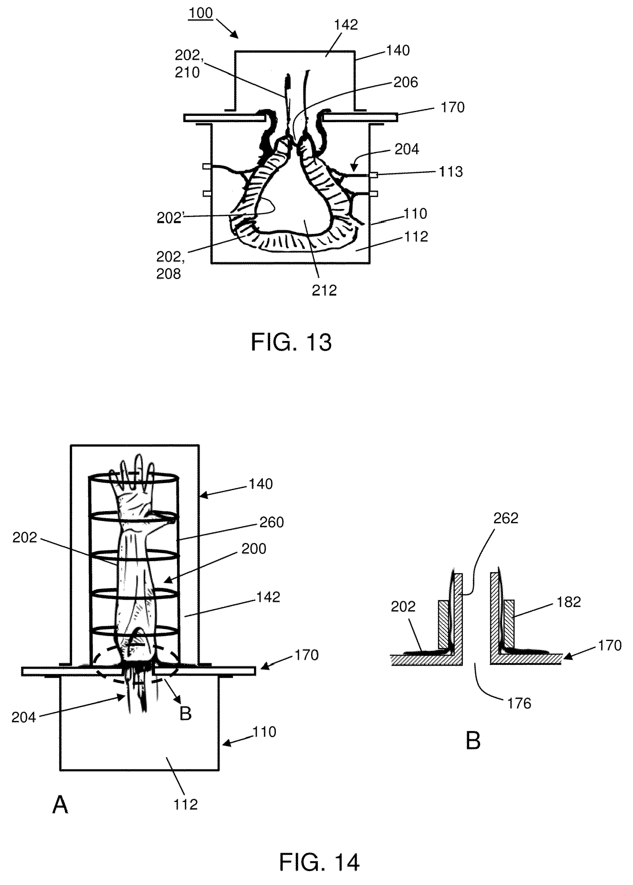

**FIG. 14** panel **B** is a section through region B of **FIG. 14** panel **A** depicting a further ancillary support structure that is hollow cylindrical projection around the aperture region and extending from the second side.

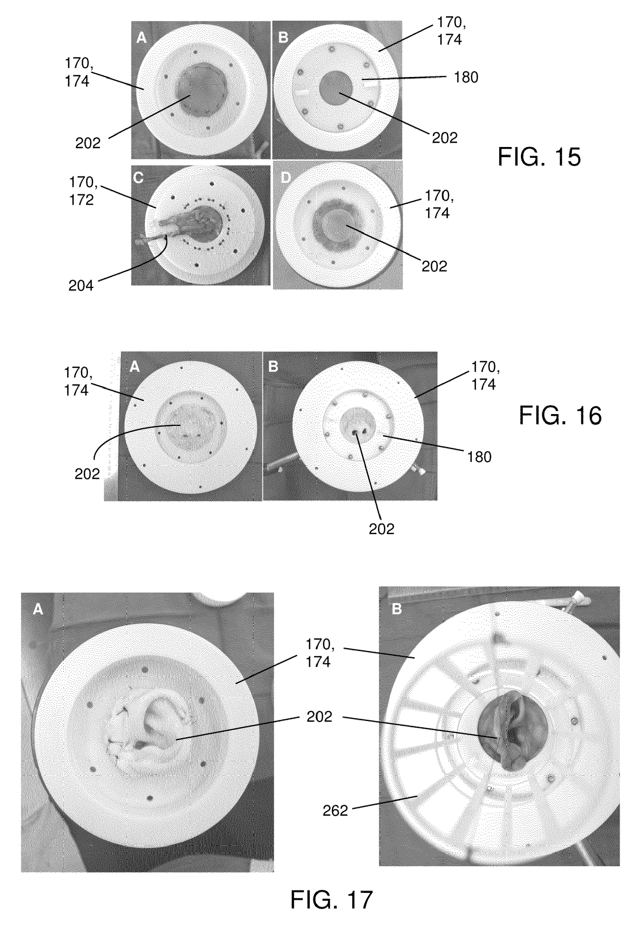

**FIG. 15** panels **A** to **D** show photographs of a vascularized structure that is a skin flap comprising a vasculated portion and a vascular pedicle mounted on a supporting element.
**FIG. 16** panels **A** and **B** show photographs of a vascularized structure (vasculated portion side) that is a nose and surrounding skin mounted on a support element, second side.
**FIG. 17** panels **A** and **B** show photographs of a vascularized structure (vasculated portion side) that is an ear and surrounding skin mounted on a support element, second side.

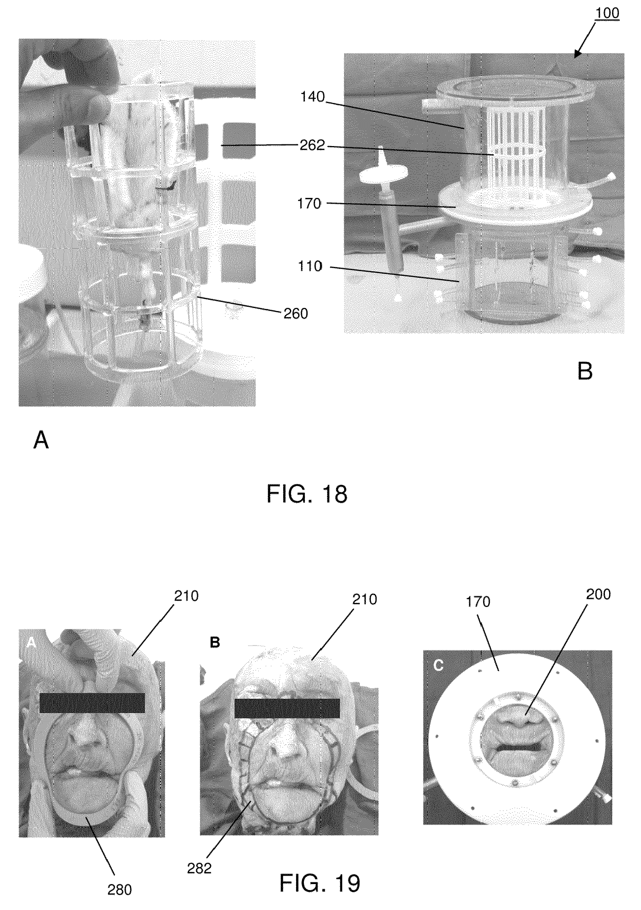

**FIG. 18** panels **A** and **B** show photographs of two ancillary support structures that are cylindrical cages.
**FIG. 19** panels **A** to **C** show photographs of harvesting a mouth and nose using a drawing template.

### Detailed description of invention

Before the present system and method of the invention are described, it is to be understood that this invention is not limited to particular systems and methods or combinations described, since such systems and methods and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1 % or less, and still more preferably +/-0.1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, *e.g*., any ≥3, ≥4, ≥5, ≥6 or ≥7 etc. of said members, and up to all said members.

All references cited in the present specification are hereby incorporated by reference in their entirety. In particular, the teachings of all references herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the present description of the invention, reference is made to the accompanying drawings that form a part hereof, and in which are shown by way of illustration only of specific embodiments in which the invention may be practiced. Parenthesized or emboldened reference numerals affixed to respective elements merely exemplify the elements by way of example, with which it is not intended to limit the respective elements. It is to be understood that other embodiments may be utilised and structural or logical changes may be made without departing from the scope of the present invention. The following detailed description, therefore, is not to be taken in a limiting sense, and the scope of the present invention is defined by the appended claims.

The present invention relates to a bioreactor for a vascularized structure. The vascularized structure comprises a vasculated portion and a vascular pedicle. In particular, the bioreactor comprises a first void space configured to receive at least part of the vascular pedicle (and inner part of the VCT if needed). The bioreactor further comprises a second void space configured to receive the at least part of the vasculated portion (other layer compartmentation). The bioreactor further comprises a support element for supporting the vascularized structure and separating the first void space from the second void space, having a body provided with a first side adjoining the first void space and a second side adjoining the second void space, a aperture region comprising one or more through apertures connecting the first side with the second side which aperture region is configured to receive the vascular pedicle therethrough. It may further comprise other inner parts from vasculated portion. The first void space may be contained in a first vessel having a first opening. The second void space may be contained in a second vessel having a second opening, or in a space formed by a flat plate and the support element, in particular an indentation therein. The bioreactor is for *in vitro* perfusion.

The bioreactor allows separation of the vasculated portion from the vascular pedicle allowing them to be maintained in different environments for optimal processing. For instance the vascular pedicle to be immersed in liquid and the vasculated portion exposed to a gaseous environment; allowing for the first time perfusion to be performed under optimal conditions.

The bioreactor has particular utility where the vascularized structure is a body part, such as skin flap, finger, limb and trunks and their subunits, ear, total face, and the like which body part is to be processed for transplantation to a patient, or *in vitro* study model. Typically, processing entails pumping of liquid through an artery or vein of the vascular pedicle, which liquid perfuses into the vasculated portion and whose composition depends upon the requirement (*e.g*. decellularization, recellularization, preservation). According to one aspect, the processing is perfusion. The perfusion may be machine perfusion. By perfusion, it is meant passing liquid into the vasculated portion via the vascular pedicle; typically liquid enters through artery(ies) and drains via vein(s). According to another aspect, the processing is direct injection. Processing may be a combination of perfusion and direct injection.

The bioreactor may be used for decellularization of a vascularized composite tissue, for recellularization of a decellularized vascularized composite tissue scaffold or for cellularization a vascularized synthetic scaffold, or for preservation of native or regenerated vascularized composite tissue.

"Decellularization" refers to a process whereby cells are removed from a vascularized composite tissue, typically by perfusion through the vascular pedicle with one or more suitable decellularization liquids, leaving a tissue scaffold that is an extracellular matrix that provides a mechanical and biochemical support. "Recellularization" refers to a process whereby a decellularized vascularized composite tissue scaffold or a vascularized synthetic scaffold is at least partially repopulated with cells, by perfusion through the vascular pedicle and/or by direction injection and/or by topical application. The cells become seeded in tissue layers. The cell may be differentiated, undifferentiated (*e.g*. stem) cells, genetically engineered cells, synthesised cells or any type of cell.

The inventors have recognised that a separation or compartmentalisation of the vascularized structure, for instance, a separation of the vascular pedicle from the vasculated portion or a separation of an inner part associated with the vascular pedicle from an outer part (see later below) that the bioreactor provides, improves handling and properties of the vascularized structure. Furthermore, support of the vascularized structure reduces mechanical stresses and damage to the delicate and often fine tissue. It protects the vascular pedicle by housing it in a vessel separate from the vasculated portion. Compared with classic organ transplantation (*e.g.* kidney, liver) where the trailing vasculature is substantial and robust, vascularized structures have typically a thin and fragile pedicle and it requires a substantially different approach. In particular where the vascularized structure includes a skin portion such as in a finger, arm or face, the vasculated portion may be contained within the first vessel under different conditions (*e.g.* gaseous) compared with the vascular pedicle (*e.g*. liquid). It is a departure from classical perfusion technology which typically utilises a single vessel. The inventors have recognised the benefit of maintaining separate environments, in particular, the need to retain the vascular pedicle in a liquid environment that may be different from skin that should not be liquid immersed for an optimum transplant tissue.

The bioreactor refers to a device that provides void spaces for the vascularized structure to provide an optimum environment for carrying out various types of procedures. It may allow a control of the composition of the environment, for instance, composition of a gas or liquid, temperature, humidity, mechanical and/or electrical stimulation. The bioreactor may be used for procedures such as perfusion, machine perfusion, preservation, and injection. The bioreactor may be used to conduct *in vitro* or *in vivo* (*e.g. in situ* regeneration of body parts) procedures. The bioreactor may be configured for use in an incubator, for instance, for control of an external environment. The bioreactor may be provided with a temperature-control jacket. The bioreactor may be provided with a fluid agitation means (*e.g.* a stirring fan or bar) for even distribution of fluid contain in one or more of the void spaces. The dimensional orientation of the bioreactor may be fixed or continuously adjustable.

The vascularized structure refers to any type of structure, including synthetic structures, that comprises a vasculated portion and a vascular pedicle. By vascularized, it is meant that the vasculated portion is provided with one or more of arteries, veins, lymphatic vessels, nerves (*e.g*. motor, sensory) which are associated with tissue types in the vasculated portion.

Examples of vascularized structures include vascularized composite tissue (VCT) (*e.g.* limb or part thereof such as an arm or finger ear, lip, tongue, face, cheek, nose), vascularized decellularized composite tissue (*e.g*. decellularized limb or part thereof such as an arm or finger ear, lip, tongue, face, cheek, nose), vascularized recellularized composite tissue (*e.g.* recellularized limb or part thereof such as an arm or finger ear, lip, tongue, face, cheek, nose), vascularized synthetic scaffold (*e.g*. 3D printed limb or part thereof such as an arm or finger ear, lip, tongue, face, cheek, nose), and organs for transplant (*e.g.* liver, kidney, heart for allotransplatation), synthetic organ.

The vascular pedicle typically comprises trailing vasculature *i.e.* an artery and/or vein for connection at one end to an external conduit for the inlet and/or outlet of perfusion fluid and which leads at the other end to the vasculated portion. It may further comprise a lymphatic vessel and/or a nerve (*e.g*. motor, sensory). The vascular pedicle may further comprise bone and other tissue such as fat (adipose) tissue, muscle, and/or tendon.

The vasculated portion is typically the substantial part of the vascularized structure and is may be vascularized composite tissue. It may equally be a decellularized vascularized composite tissue scaffold, a recellularized vascularized composite tissue scaffold, or a vascularized synthetic scaffold. The vasculated portion may comprise an organ.

Vascularized composite tissue refers to tissue having more than one tissue type associated with non-cellular material such as extracellular matrix, which tissue types are typically disposed in layers such as in an arm (containing skin, adipose tissue, tendons, muscle, bone), and ear (containing skin, adipose tissue, cartilage). Other examples of vascularized composite tissue include the face, a finger, a tongue or a lip, and the like.

Decellularized vascularized composite tissue scaffold refers to a structure, typically extracellular matrix, resulting from decellularization of vascularized composite tissue. Recellularized vascularized composite tissue scaffold refers to a structure resulting from recellularision of decellularized vascularized composite tissue. Vascularized synthetic scaffold refers to a scaffold that has been formed by a process other than decellularization, for instance, by 3D printing. The present bioreactor is suitable for use with all types of vascularized structure. The vascularized structure may be from a human or animal (*e.g*. mouse, rat, pig). It may have a synthetic origin.

In some cases vascularized structure may have an inner part and an outer part, the inner part referring to that revealed by a cross section of the vascularized structure and contained within an exterior layer such as skin or epithelium and the outer part being represented by the exterior layer. The inner part is in connection with the vascular pedicle. It is an aspect that the support element separates the inner part from the outer part; the inner part may be in fluid contact with the first void space, and the outer part may be in fluid contact with the second void space. In some cases, the inner part is contained within the first vessel, and the outer part contained within the second vessel.

The shape of vascularized structure may be essentially flat or "surfacic" wherein the vasculated portion has a height dimension that is smaller than a width dimension or has a low overall height, such as equal to or less than 2 cm. Examples of surfacic vascularized include a skin flap. Alternatively a shape of vascularized structure may be projection-like or "cylindric" (cylinder-like) wherein the vasculated portion has a height dimension that is greater than a width dimension or has a longitudinal height compared, such as greater than 2 cm. Examples of cylindric vascularized include an arm or finger. The surfacic or cylindric vascularized structure may have an influence of the second void space, in particular whether the second void space is contained in a vessel (*e.g*. for limb) or contained within a recess of the structural element (*e.g*. for a skin flap).

A vascularized structure may be typed according to the number of different exterior tissue-type surfaces *i.e.* number of different epithelia. An exterior tissue-type surface is one that can abut with the external atmosphere (*e.g*. skin) or with an interior passage or orifice or cavity (*e.g*. lip, vagina). A monophasic type of vascularized structure has predominantly one type of exterior tissue-type surface; such vascularized structure typically have an exterior tissue-type surface that is skin and includes a limb such as a finger, arm. A biphasic type of vascularized structure has predominantly two types of exterior tissue-type surfaces; for instance a lip has a skin (keratinized epithelia) exterior surface and a non-keratinized stratified squamous epithelium exterior surface that lines the oral cavity. All vascularized structures comprise the vascular pedicle.

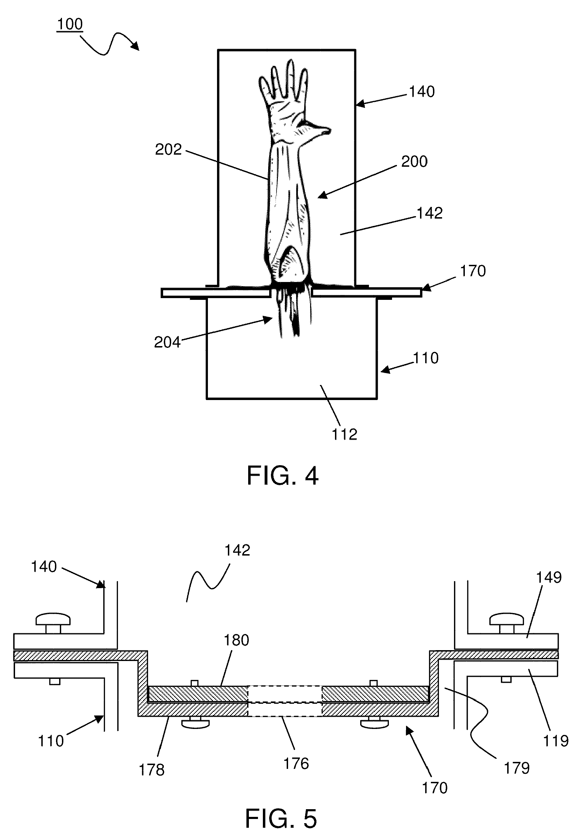

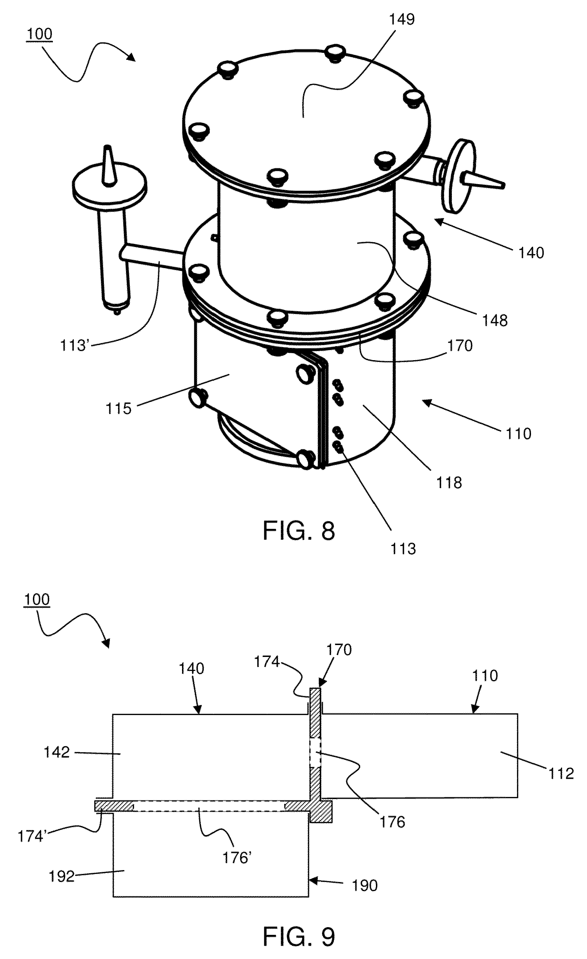

The first void space is configured to receive the vascular pedicle. The first void space is contained in a first vessel having a first opening. The first void space or first vessel is configured for holding a fluid, for instance a liquid. The first vessel may be disposed such that a longitudinal axis has an essentially vertical orientation (as shown in **FIG. 4**). The first vessel may be disposed such that a longitudinal axis has an essentially horizontal orientation (as shown in **FIG. 9**). The first vessel may have a cylindrical outer shape. The first opening may have a circular shape. The first vessel preferably has a first vessel base and a first vessel side wall extending from the first vessel base. The first vessel base may be dismountable.

The first vessel side wall and base define the first opening and first void space. The first vessel side wall may be disposed with one or more ports in fluid connection with the first void space. The ports may be configured for inlet and/or outlet of fluid, tubing, or one or more electrically conducting cables for electrical stimulation of at least part of the vasculated portion, or one or more flexible cords for mechanical stimulation (e.g. stretching). The cord may be made from a single thread or multiple threads woven together. Some of the ports may be arranged at the same or different distances from the first opening. Some of the ports may be arranged at the same or different peripheral (*e.g.* radial) positions around the side wall. The ports provide a self-contained bioreactor by permitting exchanges of fluid that control the environment of the respective void spaces, for instance, a control of temperature, gas levels (*e.g*. CO₂, NO), immersion fluid composition, of waste products and the like. It is as aspect of the invention that the bioreactor is configured for use in an incubator.

The vessel may be provided with a stimulation unit, for simulation of the vasculated portion and/or of the vascular pedicle.The stimulation unit may be provided inside and/or outside the first void space. The stimulation unit may be configured for providing mechanical stimulation; it may comprise one or more transmission elements (*e.g*. a rod, a flexible cord) for transmission of mechanical force such as a tension, compression and/or torque through the support element aperture region and into the vasculated portion. The stimulation unit may in addition or alternatively be configured for providing electrical stimulation; it may comprise one or more electrodes for transferring electrical signals to the vasculated portion for instance directly or via a nerve.

The first vessel, in particular the first vessel side wall may be disposed with a sealable access opening *i.e.* an access window to manual allow access to the first void space. The sealable access opening allows a user to connect manually tubing to the vascular pedicle for instance. It is preferably dimensioned for manual access, for instance, by fingers or hand. The sealable access opening may have any suitable shape, for instance, oval, circular or rectangular. The sealable access opening is sealable against fluid ingress by a sealing cover. The sealing cover is configured for sealing and dismountable attachment to the sealable access opening.

The first vessel may be provided with a first rim around the first opening for sealing attachment of the support element. The first rim may form an essentially planar region that sealingly engages with a co-operating region of the support element. The first rim may be provided with one or more holes or slots for attachment of a clamping device such as a nut and bolt; complementary holes or slots may be provided in the support element which align with those of the first rim.

The first void space is configured to receive the vascular pedicle, as shown for instance, in **FIG. 4****.** The first void space may be in fluid connection with an inner part of the vascularized structure. The first void space may be configured to receive at least the vascular pedicle. The first void space may be configured further to receive part of the vasculated portion, for example, in the case of a vagina and uterus as shown in **FIG. 13** wherein, a first vessel contains the vagina and uterus in the first void space, the second void space of the second vessel is in fluid connection via an orifice (vagina) with the inside of the uterus, and the support element sealingly separates the first and second void spaces by clamping around a tissue flap.

The first vessel may be made from any suitable material or materials. Typically, the first vessel is made substantially from a corrosion resistant material such as polycarbonate, glass, stainless steel or titanium. Preferably the vessel is made substantially from a transparent material such as polycarbonate or glass. The sealing cover may be made from the same material or from a different material, for instance, from stainless steel. The first vessel may be provided with a temperature regulating means, such as a temperature control jacket.

In use, the first vessel is typically provided with a liquid for immersion of the vascular pedicle during perfusion. The tubing is disposed though one or more of the ports for perfusion of the vascularized structure. The first vessel dedicated to immersion of the vascular pedicle and inner part during perfusion provides a culture medium saving and a safe pedicle handling, compared with a mono-compartmental device where the whole scaffold/organ is necessarily immersed.

The second void space is configured to be in fluid connection with at least part of the vasculated portion of the vascularized structure. The second void space may be in fluid connection with substantially all of the vasculated portion. The second void space may be in fluid connection with a phase of the vasculated portion. The second void space may be in fluid connection with the outer part of the vascularized structure. The second void space may be in fluid connection with an orifice of the vasculated portion; it may further be in fluid connection with a cavity accessed by the orifice.

The second void space may be configured to receive at least part of the vasculated portion of the vascularized structure. The second void space may be configured to receive substantially all of the vasculated portion. The second void space may be configured to receive a phase (*e.g*. skin-associated volume of scaffold) of the vasculated portion (see for instance **FIG. 4**). The second void space may be configured to receive an outer part of the vascularized structure. The second void may be configured to receive an orifice of the vasculated portion. As described above in the case of a vagina and uterus as shown in **FIG. 13** wherein, a first vessel contains the vagina and uterus in the first void space, the second void space of the second vessel is in fluid connection via an orifice (vagina) with the inside of the uterus, and the support element sealingly separates the first and second void spaces by clamping around a tissue flap. Where the vascularized structure is "surfacic", the second void space will have a corresponding low height dimension; it may be contained within a recess of the support element. Where the vascularized structure is "cylindric", the second void space will have a corresponding height dimension; it may be contained within the second vessel in communication with the support element.

The second void space may be contained in a second vessel having a second opening. The second void space may be contained contained in a gap between the support element and a covering plate; it is typical where the vascularized structure is essentially planar such as a tissue flap (see for instance **FIG. 15** panels A to D). The second void space or second vessel is configured for holding a fluid, for instance a liquid or gas. The second vessel may be disposed such that a longitudinal axis has an essentially vertical orientation (as shown in FIG. 4), an essentially horizontal orientation (as shown in **FIG. 9**), or in any orientation. The orientation may influence pressure within the vascularized structure. The second vessel may have a cylindrical outer shape. The second opening may have a circular shape. The second vessel preferably has a second vessel base and a second vessel side wall extending from the second vessel base. The second vessel base may be dismountable.

The second vessel side wall and base define the second opening and second void space. The second vessel side wall may be disposed with one or more ports in fluid connection with the second void space. The ports may be configured for inlet and/or outlet of fluid (e.g. gas), tubing, or one or more electrically conducting cables, or one or more flexible cords for mechanical stimulation (e.g. stretching). The cord may be made from a single thread or multiple threads woven together. The ports may be arranged at different distances from the second opening. Some of the ports may be arranged at the same or different peripheral (*e.g*. radial) positions around the side wall.

The second vessel may be provided with a second rim around the second opening for sealing attachment of the support element. The second rim may form an essentially planar region that sealingly engages with a co-operating region of the support element. The second rim may be provided with one or more holes or slots for attachment of a clamping device such as a nut and bolt; complementary holes or slots may be provided in the support element which align with those of the second rim.

As mentioned previously, the vessel may be provided with a stimulation unit, for simulation of the vasculated portion. The stimulation unit may be provided inside and/or outside the second void space. The stimulation unit may be configured for providing mechanical stimulation; it may comprise one or more transmission elements (*e.g*. a rod, a flexible cord) for transmission of mechanical force such as a tension, compression and/or torque to the vasculated portion. The stimulation unit may in addition or alternatively be configured for providing electrical stimulation; it may comprise one or more electrodes for transferring electrical signals to the vasculated portion for instance directly.

The second vessel may be made from any suitable material or materials. Typically, the second vessel is made substantially from a corrosion resistant material such as polycarbonate, glass, stainless steel or titanium. Preferably the second vessel is made substantially from a transparent material such as polycarbonate or glass. The second vessel may be provided with a temperature regulating means, such as a temperature control jacket.

In use, the second void space is typically provided with a fluid for immersion of the vasculated portion during perfusion. The fluid may comprise substantially a gas in some circumstances, for instance, when the vasculated portion has a skin layer in the case of a limb for example. The fluid may comprise substantially a liquid in other circumstances, for instance, when the vasculated portion contains a layer or phase that is normally in liquid contact, for instance, inside with cheek or lip. The size and/or shape of the second vessel may be adapted according to the vasculated portion, for instance, a longer second vessel might be utilised for a limb such as an arm compared with an ear.

It is an aspect of the invention that the bioreactor comprises one or more further void spaces. Each further void space is contained in a separate further vessel having an opening. In particular, where the vascularized structure is biphasic, the second vessel receives a first exterior tissue type and the further vessel receives the second tissue tissue type. As shown in **FIG. 9**, the bioreactor is disposed with three separate compartmentalised void spaces.

The support element serves to provide compartmentalisation (air and/or watertight) *i.e.* a separation of the void spaces (*e.g*. first void space from second void space), and mechanical support to the vascularized structure. The support element comprises a body provided with a first side adapted for covering the first opening, a second side adapted for covering the second opening, a aperture region comprising one or more through apertures connecting the first side with the second side which aperture region is configured for the passage of at least part of, preferably all of the vascular pedicle therethrough. The first side may be configured for sealingly covering the first opening to prevent the passage of fluid across the support element when the aperture region is sealed-off. The second side may be configured for sealingly covering the second opening to prevent the passage of fluid across the support element when the aperture region is sealed-off. Where fluidly sealing or fluidly separating is mentioned herein, it means sealing or separating to prevent to the passage of fluid

The body of the support element provides a mechanical support for the vasculated portion, for instance, the peripheral edge of the aperture region may support the weight of the vasculated portion when it is disposed on the second side. The body of the support element may further comprise one or more ancillary supporting structures, for instance a hollow cylindrical projection around the aperture region. It may extend from the first side of the support element. It may extend from the second side of the support element, as shown, for instance, in **FIG. 14B**; such ancillary supporting structure may be configured to provide upright support for the arm from within (under the skin of) the arm. Another example of an ancillary support structure is an outer cage formed, for instance, from a hollow cylinder disposed around the aperture region. It may project from the first side of the support element. It may project from the second side, as shown, **FIG. 14A**; such ancillary supporting structure may be configured to provide upright support for the arm from outside (over the skin of) the arm. Another example of an ancillary support structure is relief structure formed, for instance, from a mesh that covers the aperture region. It may project from the first side of the support element. It may project from the second side of the support element. Such ancillary supporting structure may be configured to provide support and give shape to flaccid vasculated portion such as a face. The relief-type ancillary support structure may custom made. It may be 3D printed. The ancillary supporting structure may be in permanent attachment to the support element body or may be dismountably attached to the support element body.

The body of the support element may be disposed with one or more suture anchors that provide a point for suturing the vascularized structure, preferably the vasculated portion to the support element. A suture anchor may take the form of a hook on a surface of the body or a hole that passes through the body. Suturing the vascularized structure, preferably the vasculated portion may seal the one or more apertures of the aperture region. The seal is preferably fluidic. The aperture region may be sealed-off using the vasculated portion. For instance, when the vascularized structure is an ear, the ear and skin surrounding the ear fit over the aperture region and when clamped thereover effectively seal the aperture region to prevent the passage of fluid across it. The vascularized structure in particular the vasculated portion thus functions as a gasket. The ancillary supporting structures (*e.g*. scaffolds/VCT) may be specifically harvested/designed with an extra cutaneous portion to facilitate a sealing function.

Where there are three or more void spaces, the support element may further comprise a further body containing a further aperture region which further body separates a different pair of void spaces. The further body may be separate or an extension of the body. An example of a bioreactor containing 3 void spaces is shown in **FIG. 9****.**

According to one aspect, the support element body is shaped to provide an annular peripheral gap (e.g. **FIG. 5**, 179). The gap may be present on the first side of the support element body. The gap may be created by lowering the position of the aperture region relative to the edge of the support element body; thus the support element body may be further lowered into the first void space compared with the support element body, thus forming an annular peripheral gap. The support element may be configured to position the aperture region within (*e.g*. 1 - 3 cm into) the first void space. A height of the gap may be equal to an axial distance between the first opening and the aperture region. The height of the gap may be 1 - 3 cm. The annular peripheral gap may be in fluid contact with a port present in the first side wall, allowing exchange of gases between the annular peripheral gap and the exterior environment.

The support element may further comprise a clamp configured to clamp at least part of the vasculated portion around a periphery of the aperture region thereby sealing the one or more apertures of the aperture region. The seal is preferably fluidic. The clamp may comprise a mating body that engages with the support element body around periphery of the aperture. Where the aperture region is circular, the mating body may comprise a ring (annular) shape. The mating body may be provided with one or more holes or slots for receiving a threaded bolt; the support element body may be provided with one or more threaded holes for engagingly receiving the threaded bolt. The mating body may engage with a first side or a second side of the support element body. The mating body may engage with an ancillary supporting structure of the support element body, for instance a hollow cylindrical projection around the aperture region and extending from the second side, as shown, for instance, in **FIG. 14B****.**

The support element is dismountable from the bioreactor. It may be interchanged for a support element having a different size and/or shape of aperture region. One aspect of the invention provides a kit comprising bioreactor as defined herein and a plurality of different support elements each having a different size and/or shape of aperture region. The interchangeability allow the same bioreactor to be adapted for use with a large variety of diverse vascularized structures, for instance, a face, ear, finger arm etc. or origins such as small or large animals for a same VCT type.

It is an aspect of the invention to provide a drawing template corresponding to the aperture region size and/or shape. The drawing template is used to draw an outline on the donor patient for excising vascularized structure to be harvested. Where the aperture region comprises a circular aperture, the drawing template may also be circular or annular, and define a circular region for marking the donor skin that is larger than the aperture region. The larger size allows clamping to the support structure around the periphery of the aperture region. One aspect of the invention provides a kit comprising bioreactor as defined herein and a plurality of different support elements each having a different size and/or shape of aperture region, and a plurality of drawing templates each complementary to a support element aperture region for marking a boundary for excision of the vascularized structure to be harvested such that it covers the complementary support element aperture region.

In one configuration, the support element is essentially planar having a first and second side which each sealingly covers the first and second opening respectively, more specially a respective first or second rim. The support element may be provided with one or more holes or slots for attachment of a clamping device such as a nut and bolt; complementary holes or slots may be provided in the first and/or second vessel, in particular in a first and/or second rim respectively which align with those of the support element. The support element comprises an aperture region containing a circular aperture through which the vascular pedicle passes, and an annular clamp for attaching the vasculated portion to the support element such that it seals the circular aperture.

The bioreactor may be provided with one or more additional supporting structures, configured to support the vascularized portion and/or the vascular pedicle. The additional supporting structure is preferably dismountable from the bioreactor. The additional supporting structure may be dismountably attachable to the supporting element, to the first vessel or to the second vessel. According to one aspect the additional supporting structure comprises a rod supported by the first base configured to extend through the first void space, the aperture region and at least part of the first void space; such a rod may be useful for supporting a longitudinal vasculated portion such as a limb (*e.g*. part of an arm or leg).

The present invention further provides a use of a bioreactor as described herein for perfusion of a vascularized structure. The present invention further provides a use of a bioreactor as described herein for decellularization of a vascularized composite tissue. The present invention further provides a use of a bioreactor as described herein for recellularization of a vascularized composite tissue scaffold. The present invention further provides a use of a bioreactor as described herein for preservation of vascularized composite tissue.

The present invention further comprises a method for perfusion of a vascularized structure, comprising the steps:
- providing a bioreactor as described herein,
- attaching the vascularized structure to the support element such that at least part of the vasculated portion is in fluid connection with the second void space and at least part of the vascular pedicle is disposed in the first void space,
- whereby a part of the vascularized structure is clamped over the aperture region to seal it, thereby isolating the first void space from the second void space, and
- perfusing the vascularized structure through the vascular pedicle.

The present invention further comprises a method for perfusion of a vascularized structure, comprising the steps:
- providing a bioreactor as described herein,
- attaching the vascularized structure to the support element such that at least part of the vasculated portion is disposed in the second void space and at least part of the vascular pedicle is disposed in the first void space,
- whereby a part of the vascularized structure is clamped over the aperture region to seal it, thereby isolating the first void space from the second void space, and
- perfusing the vascularized structure through the vascular pedicle.

The bioreactor may be use for perfusion of all types of vascularized structures such as
- Vascularized composite tissue Allografts (*e.g*. limb or part thereof such as an arm, ear, lip, tongue, finger, face, cheek, nose),
- Vascularized decellularized composite tissue (*e.g*. decellularized limb or part thereof such as an arm or finger, ear, lip, tongue, face, cheek, nose),
- Vascularized recellularized composite tissue (*e.g*. limb or part thereof such as an arm or finger, ear, lip, tongue, face, cheek, nose),
- Vascularized synthetic scaffold (*e.g*. 3D printed limb or part thereof such as an arm or finger, ear, lip, tongue, face, cheek, nose),
- Synthetic organs.

The vascularized structure may be of any size of volume, or any layer composition (e.g. skin, adipose tissue, muscle, cartilage, bone, mucosa), or vascularized layer. It may be used to perform decellularization, recellularization, preservation, muscular stimulation (e.g. electrical, mechanical electromechanical).

### Description of the Figures

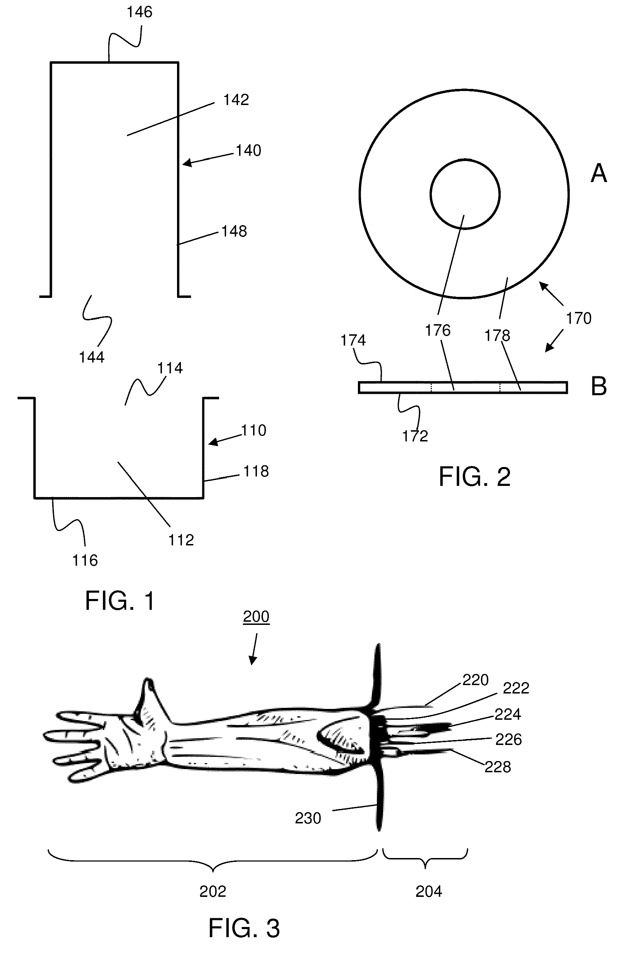

**FIG. 1** is a schematic illustration of a cross-section through a first vessel (110) and second vessel (140) of a bioreactor presently described. The first vessel (110) has a first base (116) and first side wall (118) that defines a first void space (112) and a first vessel opening (114). The second vessel (140) has a second base (146) and second side wall (148) that defines a second void space (142) and a second vessel opening (144).
**FIG. 2** panels **A** and **B** depict respectively plan and side views of a support element (170) as described herein. The support element (170) has a body (178) that is circular and disposed with a first side (172) that closes over the first opening (114) and a second side (174) that closes over the second opening (144). The body (178) is provided with an aperture region (176) connecting the first side (172) with the second side (174).
**FIG. 3** depicts a vascularized structure (200) exemplified as a part of an arm. The substantive part of the arm (hand and lower arm) form the vasculated portion (202) while elements such as arteries (229), veins (228), nerves (226) and lymphatic vessels (220) that supply the vasculated portion (202) in addition to residual muscle (222) and bone (224) form the vascular pedicle. The skin flap (230) that will form an eventual sealing gasket is also depicted.
**FIG. 4** illustrates the bioreactor (100) described herein wherein the vasculated portion (202) of the vascularized structure (200) is disposed in the second void space (142) of the second vessel (140) and vascular pedicle (204) is disposed in the first void space (112) of the first vessel (110), separated across (compartmentalised) and supported by the support element (170).
**FIG. 5** illustrates cross-sectional view of a support element (170) as described herein further comprising a clamp mating body (180) that is an annular ring co-operating with a part of the body (178) of the support element (170). The support element (170) is shown itself clamped between a first rim (119) of the first vessel (110) and a second rim (149) of the second vessel (140). An annular peripheral gap (179) is formed by lowering the aperture region (176) relative to the peripheral edge of the support element body (178).

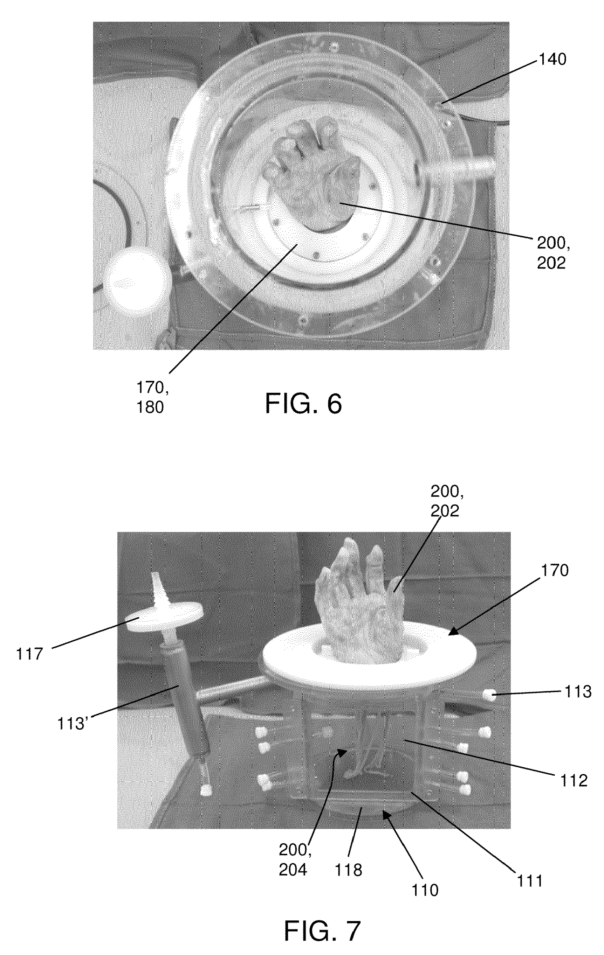

**FIG. 6** is a photograph of part of a bioreactor showing the second vessel (140) wherein a vasculated portion (202) of vascularized structure (200) that is a hand is disposed in the second void space (112). The hand is supported by the supporting structure (170) and is clamped to a body of the supporting structure (170) using a clamp mating body (180). In this example, the second vessel (140) has a removable base that has been dismounted from the second side wall.
**FIG. 7** is a photograph of part of a bioreactor showing the first vessel (110) wherein a vasculated portion (202) of vascularized structure (200) that is a hand is supported by and clamped to the supporting structure (170), and the vascular pedicle (204) of vascularized structure (200) is disposed in the first vessel (110). The first side wall (118) is provided with sealable access opening (111) for manual access to the first void space in particular to the vascular pedicle (202). The first side wall (118) is further provided with a plurality of ports (113) in fluid connection with the first void space (112). Also depicted is an upper port (113') in fluid connection with the annular peripheral gap (see FIG. 5, 179); a filter (117) is attached to retain sterility.
**FIG. 8** is an isometric view of an exemplary bioreactor (100) described herein showing the first vessel (110) having the first side wall (118) provided with sealable access opening (111) for manual access to the first void space covered with a sealing cover (115). The first side wall (118) is further provided with a plurality of ports (113, 113') in fluid connection with the first void space (112). The second vessel (140) has the second side wall (148) and a dismountable base (149). The first side wall (118) is further provided with a plurality of ports (113, 113') in fluid connection with the first void space (112). Between the first (110) and second (140) vessels is the support element (170).
**FIG. 9** is a schematic illustration of a cross-section through an exemplary bioreactor (100) as described herein comprising three void spaces (112, 132, 192), each respectively contained in a first (110), second (140) and third (190) vessel. The first void space (112) configured to receive the vascular pedicle, the second void space (142) configured to receive a part of the vasculated portion and the third void space (192) is configured to receive a remainder of the vasculated portion. A supporting element (170) separates the respective void spaces; a first body (174) of the supporting element (170) separates the first (112) and second (142) void spaces and a second body (174') of the supporting element (170) separates the second (142) and third (192) void spaces. A first aperture region (176) provided in the first body (174) is dimension to receive the vascular pedicle. A second aperture region (176') provided in the second body (174') is dimensioned to receive a part of the vasculated portion.

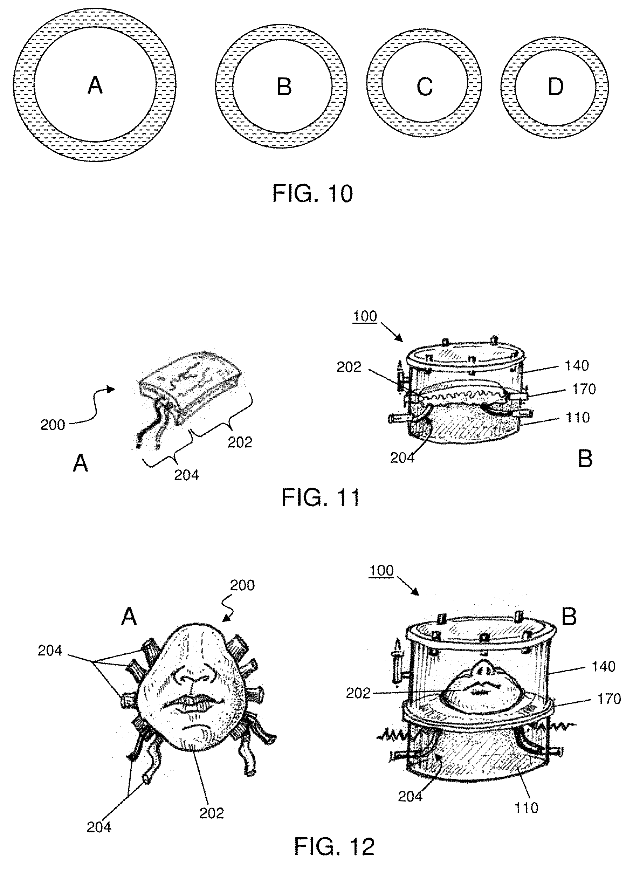

**FIG. 10** panels **A to D** depict different sizes of drawing template (large to small), each drawing template being an annular ring.
**FIG. 11** panel **A** depicts a vascularized structure (200) that is a skin flap comprising a vasculated portion (202) and a vascular pedicle (204).
**FIG. 11** panel **B** depicts the vascularized structure (200) that is the skin flap of panel **A** provided in a bioreactor (100). The vasculated portion (202) is disposed in the second vessel (140) and a vascular pedicle (204) is disposed in the first vessel (110). The respective void spaces of the first (110) and second (140) vessels are sealing separated by the support element (170).
**FIG. 12** panel **A** depicts a vascularized structure (200) that is a face comprising a vasculated portion (202) and a vascular pedicle (204).
**FIG. 12** panel **B** depicts the vascularized structure (200) that is the face of panel **A** provided in a bioreactor (100). The vasculated portion (202) is disposed in the second vessel (140) and a vascular pedicle (204) is disposed in the first vessel (110). The respective void spaces of the first (110) and second (140) vessels are sealing separated by the support element (170).
**FIG. 13** depicts a vascularized structure (200) that is a vagina and uterus comprising a vasculated portion (202, 208 (uterine), 210 (vaginal)) and a vascular pedicle (204) provided in a bioreactor. The majority of the vasculated portion (202) and the vascular pedicle (204) are disposed in the first vessel (110). A part of the vasculated portion (202) is disposed in the second vessel (140). An orifice (206) connects the uterine cavity (212) to the second void space (142) of the second vessel (140).
**FIG. 14** panel **A** depicts a vascularized structure (200) that is an arm disposed in a bioreactor (100) as shown in **FIG. 4**, together with an ancillary support structure that is a cylindrical cage (260) for mechanically supporting the exterior of the arm, akin to an exterior scaffold. Panel **B** depicts a further ancillary support structure that is hollow cylindrical projection (262) around the aperture region (176) and extending from the second side. Panel **B** further depicts a mating body (182) of a clamp, for clamping a skin flap to the support element (170) thereby sealing the aperture region (176).
**FIG. 15** shows photographs of a vascularized structure (200) that is a skin flap comprising a vasculated portion (202) and a vascular pedicle (204) mounted on a supporting element (170). Panel **A** shows the vasculated portion (202) attached to the second side (174) of the supporting element (170) using sutures. Panel **B** shows the vasculated portion (202) clamped over the aperture using a mating body (180); the skin side of the flap would be exposed to the second void space. Panel **C** shows the vascular pedicle (204) passed through the aperture region. Panel **D** shows the vasculated portion (202) after removal of the mating body (180).
**FIG. 16** shows photographs of a vascularized structure (vasculated portion (202) side) that is a nose and surrounding skin mounted on a support element (170), second side (174). Panel **A** is prior to clamping, in Panel **B**, the clamp mating body (180) has been applied.
**FIG. 17** shows photographs of a vascularized structure (vasculated portion (202) side) that is an ear and surrounding skin mounted on a support element (170), second side (174). Panel **A** is prior to clamping, in Panel **B**, the clamp mating body (180) has been applied, and further an ancillary support structure that is a cylindrical cage (262) is depicted.
**FIG. 18** shows photographs of two ancillary support structures that are cylindrical cages (260, 262). In Panel **A**, an inner cage (260) is shown, Panel **B**, an outer cage (262) is shown mounted in the bioreactor (100).
**FIG. 19** show photographs of harvesting a mouth and nose using a drawing template. In Panel **A**, a drawing template (280) this is an annular ring is applied to the face region of the head (210) so that the hole of the ring covers the nose and mouth. Lines (282) are drawn on the facing using the inner and outer edge of the template as a guide (Panel **B**). The vascularized structure (200) is harvested and placed over the aperture region of the support element (170) (Panel **C**).

## Claims

1. A bioreactor (100) for a vascularized structure (200) comprising a vasculated portion (202) and a vascular pedicle (204), the bioreactor (100) comprising:
- a first void space (112) configured to receive the vascular pedicle (204) optionally contained in a first vessel (110) having a first opening (114),
- a second void space (142) in fluid contact with at least part of the vasculated portion (202), and
- a support element (170) for supporting the vascularized structure (200) and fluidly separating the first void space (112) from the second void space (142), comprising an aperture region (176) comprising one or more apertures configured to receive at least part of the vascular pedicle (204).

2. The bioreactor (100) according to claim 1 wherein:
- the support element (170) comprises a body provided with a first side (172) adjoining the first void space (112) and a second side (174) adjoining the second void space (142), and wherein the aperture region (176) connects the first side (172) with the second side (174), and
- the second void space (142) is contained in a second vessel (140) having a second opening (144) or contained in a gap between the support element (170) and a covering plate.

3. The bioreactor (100) according to claim 1 or 2, wherein the support element (170) further comprises a clamp or more suture anchors configured to fixedly attach at least part of the vasculated portion (202) around a periphery of the aperture region (176) thereby fluidly sealing the aperture region (176).

4. The bioreactor (100) according to claim 2 or 3, wherein the first (110) vessel and second vessel (140) or covering plate are each dismountably attachable to the support element (170).

5. The bioreactor (100) according to any of claims 1 to 4, wherein the first vessel (110) is further disposed with a sealable access opening (111), for manual access to the vascular pedicle (204).

6. The bioreactor (100) according to any of claims 1 to 5, wherein the first vessel (110) is further disposed with one or more ports (113) in fluid connection with the first void space (112) for inlet and/or outlet of fluid, of one or more electrically conducting cables, or of one or more flexible cords, optionally some ports being arranged at different distances from the first opening (114) or a different peripheral positions around first vessel.

7. The bioreactor (100) according to any of claims 2 to 6, wherein the second vessel (140) is further disposed with one or more ports (113) in fluid connection with the second void space (142) for inlet and/or outlet of fluid, of one or more electrically conducting cables, or of one or more flexible cords, optionally some ports on the second vessel (140) being arranged at different distances from the second opening (114) or a different peripheral positions around second vessel.

8. The bioreactor (100) according to any of claims 1 to 6, wherein the support element (170) is configured to position the aperture region (176) within the first void space (112), and optionally to provide a gap between a wall (118) of the first vessel (110) and the support element (170), optionally the gap having an annular form.

9. The bioreactor (100) according to any of claims 1 to 7, further comprising a third void space (192) separated from the first (112) or second (142) void space by the support element (170), configured to receive at least a part of the vasculated portion (202) not received by the received by the second void space (142).

10. Use of a bioreactor (100) according to any of claims 1 to 9, for perfusion of a vascularized structure (200), for decellularization of a vascularized composite tissue, recellularization of a vascularized composite tissue scaffold or for preservation of vascularized composite tissue.

11. Use according to claim 10, wherein:
- the first void space (112) is configured to contain liquid, and the second void space (142) is configured to contain a gaseous atmosphere or *vice versa,* or
- the first void space (112) and the second void space (142) are each configured to contain a liquid, or
- the first void space (112) and the second void space (142) are each configured to contain a gaseous environment.

12. A method for perfusion of a vascularized structure (200), comprising the steps:
- providing a bioreactor (100) according to any of claims 1 to 9,
- attaching the vascularized structure (200) to the support element (170) such that at least part of the vasculated portion (202) is in fluid connection with the second void space (142) and at least part of the vascular pedicle (204) is disposed in the first void space (112),
- whereby a part of the vascularized structure (200) is sutured and/or clamped over the aperture region (176) to seal it, thereby isolating the first void space (112) from the second void space (142), and
- perfusing the vascularized structure (200) through vasculature of vascular pedicle (204).

13. A kit comprising a bioreactor (100) according to any of claims 1 to 9, wherein there are at least two interchangeable support elements (170), each having a different aperture region (176) size and/or shape.

14. The kit according to claim 13, further comprising at least two different drawing templates, one for each different support element (170) for marking skin of a donor for harvesting such that the size and shape of the skin harvested is suitable for suturing and/or clamping over the corresponding aperture region (176) size and/or shape of the support element (170).

15. The bioreactor (100) according to any of claims 1 to 9, the use according to claim 10 or 11, the method according to claim 12 or the kit according to claim 13 or 14 wherein the vascularized structure (200) is a vascularized composite tissue or a vascularized composite tissue scaffold.
